# EUROPÄISCHE PATENTSCHRIFT

(11) **EP 2 491 367 B1**
(45) Veröffentlichungstag und Bekanntmachung des Hinweises auf die Patenterteilung: **24.07.2013**
(21) Anmeldenummer: 10795610.4
(22) Anmeldetag: 21.10.2010
(51) Int. Cl.: G01N 29/14, G01N 3/32, G01N 29/46

(54) **VERFAHREN ZUR BESTIMMUNG DER WEICHHEIT VON TISSUEPAPIER**
METHOD FOR DETERMINING THE SOFTNESS OF TISSUE PAPER
PROCÉDÉ PERMETTANT DE DÉTERMINER LA SOUPLESSE DE PAPIER À USAGE SANITAIRE ET DOMESTIQUE

(30) Priorität: 23.10.2009 DE 102009051686
(43) Veröffentlichungstag der Anmeldung: 29.08.2012
(73) Patentinhaber: Fraunhofer-Gesellschaft zur Förderung der angewandten Forschung e.V., 80686 München (DE); Technische Universität Dresden, 01069 Dresden (DE)
(72) Erfinder: TSCHÖPE, Constanze, 01558 Grossenhain (DE); WOLFF, Matthias, 02826 Görlitz (DE); BORCHERS, Bernhard, 01809 Heidenau (DE)
(74) Vertreter: Pfenning, Meinig & Partner GbR
(86) Internationale Anmeldenummer: PCT/DE2010/001269
(87) Internationale Veröffentlichungsnummer: WO 2011/047673

(56) Entgegenhaltungen:
- WO-A1-2007/093484
- US-A1- 2008 053 231
- Liu J.: "Effects of Chemical Additives on the Light Weight Paper, Dissertation", Internet Article, 2004, Seiten I-XX,1-308, XP002646901, Georgia Gefunden im Internet: URL:http://smartech.gatech.edu/bitstream/h andle/1853/7626/liu_jin_200412_phd.pdf;jse ssionid=80239D83659496B17C0FDD30A696C78E.s mart2?sequence=1 [gefunden am 2011-07-01]
- TSCHÖPE C ET AL: "Classification of non-speech acoustic signals using structure models", 2004 IEEE INTERNATIONAL CONFERENCE ON ACOUSTICS, SPEECH, AND SIGNAL PROCESSING, Bd. 5, 2004, Seiten V-653-V-656, XP002646902, IEEE PISCATAWAY, NJ, USA ISBN: 0-7803-8484-9
- YI E ET AL: "A fabric sound evaluation system for totally auditory-sensible textiles", TEXTILE RESEARCH JOURNAL, Bd. 72, Nr. 7, Juli 2002 (2002-07), Seiten 638-644, XP002646903, TEXTILE RESEARCH INSTITUTE US
- HOLLMARK HOLGER ET AL: "MEASUREMENT OF TISSUE PAPER SOFTNESS: A LITERATURE REVIEW", NORDIC PULP AND PAPER RESEARCH JOURNAL, Bd. 19, Nr. 3, 1. Januar 2004 (2004-01-01) , Seiten 345-353, XP008079233, STOCKHOLM, SE ISSN: 0283-2631, DOI: 10.3183/NPPRJ-2004-19-03-P345-353
- Tschöpe C. et al: "Instrumentelle Bestimmung der Weichheit von Tissueprodukten", Internet Article, 2010, XP002646904, Gefunden im Internet: URL:www.dgaqs.de/forum2010/prf/TB-03_Tsch% C3%B6pe.pdf [gefunden am 2011-07-01]

## Beschreibung

Die Erfindung betrifft ein Verfahren zur Bestimmung der Weichheit von Tissuepapier. Dies können so genannte Hygienepapiere, wie z.B. Taschen-, Kosmetik-, Küchen-, Handtücher oder Toilettenpapier, sein.

An die Weichheit solcher Papiere werden seitens der Verbraucher hohe Anforderungen gestellt. Im Gegensatz zu anderen Materialien und auch anderen höher festen Papieren besteht bisher keine Möglichkeit hierfür ein objektives und quantitatives Maß für die jeweilige Weichheit anzugeben, wie dies beispielsweise für die Festigkeit oder das Wasseraufnahmevermögen möglich ist.

Als Methode für die Bewertung der Weichheit von Tissuepapier hat sich in der Vergangenheit ein subjektiv geprägtes Bewertungssystem weitestgehend durchgesetzt, das als Paneltest bezeichnet wird. Hierbei werden entsprechende Proben von geschulten Testpersonen haptisch manuell getestet. Die Proben werden dabei mit der Fingerkuppe ertastet, von Hand gefaltet und geknüllt. Der jeweilige subjektiv von der Testperson ermittelte Eindruck kann auch durch Vergleich unterschiedlicher Proben entsprechenden Weichheitsklassen zugeordnet werden. Die Zuordnung kann durch geeignete mathematische Auswahlverfahren in die jeweiligen Weichheitsklassen unter Berücksichtigung aller Ergebnisse der bei einem Test mitwirkenden Testpersonen erfolgen.

Bei jedem einzelnen in dieser Form durchzuführenden Test ist eine ausreichend große Anzahl an geeigneten Testpersonen erforderlich, um repräsentative Ergebnisse erhalten zu können. Es liegt auf der Hand, dass dies nur in mehr oder weniger großen Abständen durchgeführt werden kann und eine herstellungsbegleitende Prüfung oder gar ständige Qualitätskontrolle in dieser Form nicht möglich ist. Insbesondere kann so kein Vergleich unterschiedlicher Produkte von unterschiedlichen Herstellern vorgenommen werden.

Es hat daher in der Vergangenheit Versuche gegeben solche Tests durch den Einsatz geeigneter Prüftechnik zu ermöglichen.

So ist von S. Kawabata in "Testing the tactile properties of tissue and nonwovens"; Borch, Mark, Habeger, Handbook of physical testing of paper; Vol. 2, Marcel Dekker Inc. New York, Basel; S. 505 - 529 darauf hingewiesen, dass viele Parameter bestimmt werden können, um eine Bewertung vornehmen zu können. So sollen die Zug- und Scherfestigkeit, Biegesteifigkeit, flächenbezogene Masse, Dicke, Kompressibilität und Oberflächenbeschaffenheit bestimmt werden. Für diese doch sehr unterschiedlichen Parameter und Messgrößen ist der Einsatz einer entsprechenden Anzahl unterschiedlicher Messvorrichtungen erforderlich. Demzufolge sind der gerätetechnische sowie der zeitliche Aufwand für Messung und Auswertung erheblich, so dass auch damit keine praktikable universell einsetzbare technische Lösung gegeben ist.

Außerdem ist in DE 102 28 923 A1 eine Prüfvorrichtung zur Bestimmung der mechanischen Eigenschaften flächenhafter Materialien bekannt. Dabei sollen Weg- und Kraftverläufe bestimmt werden. Inwiefern die eigentliche Auswertung erfolgen soll, bleibt jedoch offen.

Die Dissertation "Liu J.: Effects of Chemical Additives on the Light Weight Paper", Georgia Institute of Technology, Oktober 2004, beschreibt ein Verfahren zur Bestimmung der Weichheit von Tissuepapier, bei dem Proben mechanisch mit Druck und/oder Zugkräften mit jeweils gleichen Parametern beaufschlagt werden und die Proben nach Vergleich mit entsprechenden Signalen für manuell geprüfte Proben Weichheitsklassen zugeordnet werden. Die Auswertung der Ergebnisse, d.h. Zuordnung der physikalischen Messwerte zu den subjektiven Zuordnungen zu Weichheitsklassen erfolgt gemäß Regression.

Ein Verfahren und eine Vorrichtung zur Bestimmung der Weichheit von Hygienepapieren und Textilien sind in WO 2007/093484 A1 beschrieben. Damit soll eine Kraftwirkung mit einer Relativbewegung auf eine Probe ausgeübt und die dabei auftretenden Schwingungen/Geräusche mit einem Schwingungssensor erfasst werden.

Die erfassten Schwingungen sollen mittels einer Schwingungsanalyse unter Ermittlung eines Schallspektrums oder Frequenzbandes ausgewertet und dabei jedem Schallspektrum oder Frequenzband eine bestimmte Weichheit der jeweiligen Probe zugeordnet werden. Hierfür sollen die Schallintensität und/oder der Schallpegel und/oder die Frequenzen des Schalldrucks in Bereichen des Schallspektrums und/oder des Frequenzbandes genutzt werden, um daraus eine komplexe Kennzahl zu berechnen, die mit der Weichheit der Probe korreliert. Es werden bestimmte vorgebbare Bereiche des Schallspektrums genutzt. Bei der Auswertung sollen Referenzmerkmale berücksichtigt werden.

Aufgabe der Erfindung ist es, die Weichheit von Proben aus Tissuepapier in einfacher Form und erhöhter Aussagekraft zu bestimmen.

Erfindungsgemäß wird diese Aufgabe mit einem Verfahren, das die Merkmale des Anspruchs 1 aufweist, gelöst. Vorteilhafte Ausgestaltungen und Weiterbildungen der Erfindung können mit in untergeordneten Ansprüchen bezeichneten Merkmalen erreicht werden.

Bei dem erfindungsgemäßen Verfahren zur Bestimmung der Weichheit von Tissuepapier werden Proben von Tissuepapieren mechanisch mit Druck- und/oder Zugkräften mit jeweils gleichen Parametern beaufschlagt. Dabei werden dadurch emittierte Schallwellen mit mindestens einem Detektor erfasst. Die so erfassten Messsignale werden dann einer Mustererkennung unterzogen.

Hierfür werden einmal Proben, die vorab manuell durch subjektiv, haptisch von Testpersonen geprüft und in Weichheitsklassen eingeteilt worden sind (Paneltest), und außerdem unabhänige vorab manuell ungeprüfte Proben genutzt. Die unabhängig vorab manuell ungeprüften Proben werden dann nach Vergleich von Mustern der vorab manuell geprüften Proben einer Weichheitsklasse zugeordnet.

Dabei kann die Anzahl der manuell vorab geprüften Proben deutlich kleiner als die Anzahl der untersuchten vorab ungeprüften Proben sein. Besonders günstig ist es, wenn vorab manuell geprüfte Proben zyklisch in Zeitabständen einer erfindungsgemäßen Bestimmung unterzogen werden, da dadurch die Anzahl an vergleichbaren Mustern von Schallmesssignalen, die bereits bestimmten Weichheitsklassen zugeordnet worden sind, ansteigt und so die Aussagekraft und die Wahrscheinlichkeit der Richtigkeit der Bestimmungsergebnisse über die Zeit erhöht werden können.

Die Proben können mechanisch bewegt werden, wie dies auch beim manuellen Paneltest erfolgt. Die jeweilige Krafteinwirkung sollte aber jeweils in gleicher Form erfolgen.

Es hat sich heraus gestellt, dass gute Ergebnisse erreicht werden können, wenn Zugkräfte an einer Probe wirken, die zum Reißen führen. Während des Reißens werden Schallwellen emittiert, die mit mindestens einem Detektor (z.B. Mikrofon, Körperschallsensor) detektiert werden können. Bevorzugt wird dabei an den Proben ein Anriss ausgebildet. Dies kann eine definierte Aussparung an einem Rand einer Probe sein. Diese kann beispielsweise ausgestanzt worden sein. Die Erfassung der Schallwellenmesssignale erfolgt bevorzugt erst beim Weiterreißen.

Während dieses Vorgangs, der zum Reißen von Proben führt, sollten konstante Bedingungen eingehalten werden, was insbesondere die Temperatur, relative Feuchte, die Anordnung des/der Detektors/Detektoren in Bezug zur Probe und die Art und Weise des Zugkraftangriffs betrifft.

Die beim Reißen wirkenden Zugkräfte sollten zeitaufgelöst erfasst und bei der Bestimmung der Weichheit mit berücksichtigt werden. Die Aussagen des zeitlichen Kraftverlaufs können dabei mitberücksichtigt werden.

Für die Mustererkennung sollten für die Schallmesssignale der jeweiligen Proben Merkmale bestimmt und diese Merkmale dann miteinander verglichen werden. Dies kann beispielsweise durch eine Kurzzeitspektralanalyse oder eine analoge Vorgehensweise erreicht werden.

Da bei den erfassten Schallmesssignalen typische Ausprägungen und Abfolgen vorkommen, können diese mit Verfahren der Signalanalyse, der Merkmalextraktion, - kompression und statistischen Mustererkennung zur Erstellung von Modellen genutzt werden. Dies kann auch bei unbekannten Schallmesssignalverläufen oder Mustern erfolgen.

Die Merkmale sollten nach bestmöglicher Eignung für die Mustererkennung ausgewählt werden. Hierfür können u.a. eine Kurzzeitspektralanalyse (Leistungsdichtespektrum), Wavelet-Transformation, Kurzzeit-Cepstrum, Pegelkreuzungsghistogramme, Kurzzeit-Fourier-t-Transformation oder pseudoakustisch motivierte Verfahren, wie beispielsweise Mel-skalierte Cepstrahlkoeffizienten (MFCC) eingesetzt werden.

Die Kompression der Merkmale kann automatisch durch statistische Verfahren, z.B. eine Hauptkomponentenanalyse (HKA) oder lineare Diskriminanzanalyse (LDA), erreicht werden.

Der Vergleich dieser Merkmale der jeweiligen Proben kann dann mit einem mathematischen Modell, das ausgewählt ist aus dem Gaussian Mixture Modell, und dem Hidden Markov Modell. worden sind.

Diese Modelle sind lernfähig und können problemunabhängig angewandt werden. Sie benötigen wenig oder gar kein A-priori-Wissen, sind skalierbar und echtzeitfähig. Es ist eine Kombination von Schallmesssignalen, die von mehreren Detektoren an einer Probe erfasst worden sind, möglich.

Bei einer Merkmalextraktion können aus dem Schallmesssignalverlauf in gleichmäßigen Abständen einzelne Merkmalvektoren und somit eine Merkmalvektorfolge gebildet werden. Es können dabei nicht nur die räumlichen Eigenschaften, sondern auch die zeitlichen Strukturen der ausgewählten Spektren berücksichtigt werden. Eine kurze Folge von so ermittelten bzw. ausgewählten Merkmalvektoren kann für eine Zuordnung der jeweiligen Weichheit genutzt werden. Die ausgewählten Merkmalvektoren können im Modell weiter verarbeitet werden. So können beim Hidden-Markov-Modell Verteilungsdichtefunktionen beschrieben werden.

Dabei kann die Modellierung der ausgewählten Merkmalvektoren, also von Schallmesssignalstrukturen, als Markov-Kette erster Ordnung erfolgen. Jeder ermittelte Weichheitszustand einer Probe tritt mit einer bestimmten Wahrscheinlichkeit auf. Die jeweilige Weichheit muss aber nicht bekannt sein, da die Modelle lernfähig sind.

Mit der Erfindung ist es im Gegensatz zum Stand der Technik, bei dem die Bestimmung rein messtechnisch durchgeführt wird, möglich, die Weichheit von Tissuepapier zu bestimmen, indem das Urteil von Testpersonen automatisch nachvollzogen werden kann. An die Stelle der Bestimmung eines Maßes können also Paneltests nachvollzogen werden. Dies erfolgt aber nicht heuristisch, sondern durch ein Maschinenlernverfahren. Das Urteilsvermögen von Testpersonen kann dabei erlernt werden.

Nachfolgend soll die Erfindung beispielhaft näher erläutert werden.

Dabei zeigt:

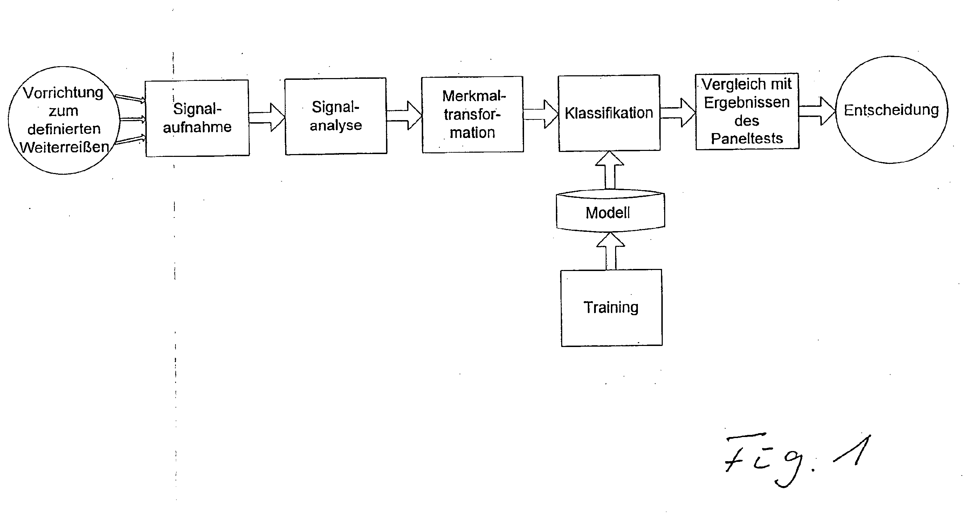

Figur 1 ein Flussdiagramm für den Ablauf der Bestimmung der Weichheit von Tissuepapier.

Mit einer Vorrichtung zur Messung der Durchreißfestigkeit nach Elmendorf wurden 50 Proben von fünf Typen von Kosmetiktüchern (A bis F) mit unterschiedlicher Weichheit zerrissen. Die dabei emittierten Schallwellen wurden mit einem Mikrofon erfasst und als Zeitreihe gespeichert. Die Einordnung in die Weichheitsklassen A bis F erfolgte dabei vorab mittels Paneltest, der von Testpersonen durchgeführt worden ist.

In einem Kreuzvalidisierungsverfahren wurde aus den gespeicherten Schallmesssignalen von 40 Proben (Lerndatensatz) je ein Hidden-Markov-Modell für jede Weichheitsklasse A bis F automatisch erstellt. Die gespeicherten Schallmesssignale der restlichen 10 Proben (Testdatensatz) wurden für jede Weichheitsklasse mit Hilfe dieser Modelle erkannt. Dieses Verfahren wurde für alle Proben fünfmal wiederholt, so dass jede Aufzeichnung genau einmal zur Klassifikation verwendet wurde. Es konnten mit dieser kleinen Anzahl an Proben und durchgeführten Untersuchungen, mit einem sehr einfachen Messaufbau, ohne Klimatisierung, spezielle Anpassung des Messgeräts und auch ohne Optimierung der Messung der emittierten Schallwellen, bei in keiner Weise problemangepassten Schallmesssignalverarbeitung und Mustererkennung von 187 Proben aus der Gesamtprobenanzahl von 250 richtig in die jeweilige Weichheitsklasse zugeordnet werden.

Nachfolgend soll auf das in Figur 1 gezeigte Flussdiagram näher eingegangen werden.

Es werden mit mehreren Mikrofonen oder anderen geeigneten Detektoren beim Reißen von Proben aus Tissuepapier emittierte Schallwellen erfasst und einer Signalaufnahme zugeführt. Dabei wurden Schallmesssignale beim Weiterreißen ausgehend von einem vorab an den Proben ausgebildeten definierten Anriss erfasst.

Da nicht alle einzelnen zeitaufgelöst erfassten Schallmesssignale wegen redundanter und irrelevante Informationen repräsentierende Einzelmesssignale genutzt werden müssen, werden die Schallmesssignale einer Signalanalyse unterzogen. Mit geeignete Merkmale aufweisenden Schallmesssignalverläufen wurde zur weiteren Reduzierung der Redundanz eine Merkmaltransformation durchgeführt. Dieser Schritt ist jedoch nicht zwingend erforderlich.

Für eine nachfolgend durchzuführende Klassifikation werden ein oder mehrere Schallmesssignalmodelle gebildet.

Das Training oder der Lernprozess erfolgt über die Zeit, mit ansteigenden in einer Datenbasis enthaltenen Schallmesssignalverläufen und in Kenntnis von parallel durchgeführten Paneltests, automatisch.

Die ausgewerteten Daten werden dann mit den durch Testpersonen bei Paneltests ermittelten Ergebnissen verglichen, um eine letztendliche Entscheidung zu treffen, bei der die jeweiligen Proben einer Weichheitsklasse zugeordnet werden. Die Weichheit von Proben aus Tissuepapier soll in einfacher Form und mit erhöhter Aussagekraft bestimmt werden können.

## Patentansprüche

1. Verfahren zur Bestimmung der Weichheit von Tissuepapier, bei dem Proben mechanisch mit Druck- und/oder Zugkräften, bei jeweils gleicher Krafteinwirkung beaufschlagt und dabei emittierte Schallwellen mit mindestens einem Detektor erfasst werden; dabei
die erfassten Schallmesssignale einer Mustererkennung unterzogen werden, wobei dies für Proben, die vorab manuell durch subjektive, haptische Prüfung von Testpersonen und Einteilung der jeweiligen Proben in Weichheitsklassen und unabhängige vorab manuell ungeprüfte Proben durchgeführt wird und die unabhängig vorab manuell ungeprüften nach Vergleich von Mustern der vorab manuell geprüften Proben einer Weichheitsklasse zugeordnet werden;
wobei der Vergleich der Merkmale der Schallmesssignale der Proben mit einem mathematischen Modell, das ausgewählt ist aus dem Gaussian Mixture Modell, und dem Hidden Markov Modell, durchgeführt wird.

2. Verfahren nach Anspruch 1, **dadurch gekennzeichnet, dass** die Proben definiert ausgehend von einem bereits vorhandenen Anriss durch an Rändern wirkende Zugkräfte auseinander gerissen werden und die dabei emittierten Schallwellen detektiert und ausgewertet werden.

3. Verfahren nach Anspruch 1 oder 2, **dadurch gekennzeichnet, dass** die beim Reißen wirkenden Kräfte zeitaufgelöst erfasst und bei der Bestimmung der Weichheit, durch Aussagen des zeitlichen Kraftverlaufs berücksichtigt werden.

4. Verfahren nach einem der vorhergehenden Ansprüche, **dadurch gekennzeichnet, dass** die Bestimmung von Merkmalen der Schallmesssignale mittels Kurzzeitspektralanalyse durchgeführt wird.

## Claims

1. A method for determining the softness of tissue paper, in which samples are acted upon mechanically with pressure forces and/or tensile forces, with the same action of force each time, and sound waves emitted in so doing are detected with at least one detector; in so doing
the detected sound measurement signals are subjected to pattern recognition, this being carried out for samples which in advance manually by subjective, haptic testing by test persons and dividing the respective samples into softness classes and independent samples manually untested in advance, and the ones independently manually untested in advance after comparison of patterns of the samples tested manually in advance being associated with a softness class,
the comparison of the features of the sound measurement signals of the samples being carried out with a mathematical model which is selected from the Gaussian mixture model and the hidden Markov model.

2. A method according to Claim 1, **characterised in that** the samples are torn apart in defined manner starting from an already-present incipient tear by tensile forces which act on edges, and the sound waves emitted in so doing are detected and evaluated.

3. A method according to Claim 1 or 2, **characterised in that** the forces which act during tearing are detected in time-resolved manner and are taken into account in the determination of the softness by evidence of the force progression over time.

4. A method according to one of the preceding claims, **characterised in that** the determination of features of the sound measurement signals is carried out by means of short-time spectral analysis.

## Revendications

1. Procédé pour déterminer la douceur d'un papier tissu, dans lequel des échantillons sont soumis mécaniquement à des forces de pression et/ou de traction, pour une action de force respectivement identique, et des ondes sonores émises à cette occasion sont détectées avec au moins un détecteur ; en outre
les signaux de mesure sonores détectés sont soumis à une reconnaissance de formes, cela étant effectué pour des échantillons, qui ont été au préalable soumis manuellement à des tests de toucher subjectifs par des sujets d'expériences et classés respectivement dans des classes de douceur et pour des échantillons indépendants non testés manuellement au préalable, et les échantillons indépendants non testés manuellement au préalable sont affectés à une classe de douceur après comparaison des formes d'échantillons testés manuellement au préalable ;
la comparaison des caractéristiques des signaux de mesure sonores des échantillons étant effectuée avec un modèle mathématique, qui est sélectionné parmi le modèle de mélanges gaussiens et le modèle de Markov caché.

2. Procédé selon la revendication 1, **caractérisé en ce que** les échantillons sont déchirés de façon définie par des forces de traction agissant sur des bords, à partir d'une amorce de déchirure préexistante, et les ondes sonores émises à cette occasion sont détectées et analysées.

3. Procédé selon la revendication 1 ou 2, **caractérisé en ce que** les forces agissant lors de la déchirure sont enregistrées avec une résolution temporelle et sont prises en compte lors de la détermination de la douceur par des constatations de l'évolution des forces dans le temps.

4. Procédé selon l'une quelconque des revendications précédentes, **caractérisé en ce que** la détermination de caractéristiques des signaux de mesure sonores est effectuée au moyen de l'analyse spectrale de courte durée.
